# FASCICULE DE BREVET EUROPEEN

(11) **EP 1 088 099 B1**
(45) Date de publication et mention de la délivrance du brevet: **25.02.2004**
(21) Numéro de dépôt: 00920813.3
(22) Date de dépôt: 17.04.2000
(51) Int. Cl.: C12Q 1/04

(54) **PROCEDE DE DETERMINATION DE L'EXISTENCE D'UNE ACTIVITE BIOLOGIQUE DE MICRO-ORGANISMES**
VERFAHREN ZUR BESTIMMUNG EINER BIOLOGISCHEN AKTIVITÄT VON MIKROORGANISMEN
METHOD FOR DETERMINING THE EXISTENCE OF A BIOLOGICAL ACTIVITY OF MICROORGANISMS

(30) Priorité: 15.04.1999 FR 9904731
(43) Date de publication de la demande: 04.04.2001
(73) Titulaire: Etat Français représenté par le Délégué Général pour l' Armement, 00457 Armées (FR)
(72) Inventeur: BOISRAYON, Michel, F-83330 Le Beausset (FR); SCARPITTA, Alain, F-83200 Toulon (FR); DENUNZIO DEJUGNAC, Corinne, 83500 La Seyne Sur Mer (FR); BARSOTTI, Nathalie, F-83140 Six Fours (FR); MATHERON, Robert, F-13005 Marseille (FR); LE PETIT, Jean, F-13190 Allanch (FR); ANGLADE, Jean-Yves, F-13004 Marseille (FR)
(86) Numéro de dépôt international: PCT/FR2000/000988
(87) Numéro de publication internationale: WO 2000/063428

(56) Documents cités:
- EP-A- 0 184 260
- US-A- 4 321 322
- US-A- 4 898 816
- US-A- 5 824 494
- DATABASE CHEMABS [en ligne] CHEMICAL ABSTRACTS SERVICE, COLUMBUS, OHIO, US; HAMILTON, WILLIAM ALLAN ET AL: "Effects of high electric fields on microorganisms. II. Mechanism of action of the lethal effect" retrieved from STN Database accession no. 68:36989 XP002127047 & BIOCHIM. BIOPHYS. ACTA (1967), 148(3), 789-800,
- PATENT ABSTRACTS OF JAPAN vol. 1999, no. 10, 31 août 1999 (1999-08-31) & JP 11 127891 A (MATSUSHITA ELECTRIC IND CO LTD), 18 mai 1999 (1999-05-18)

## Description

La présente invention concerne un procédé permettant de déterminer si des micro-organismes ont conservé ou non une activité biologique.

L'intensité du courant de fuite généré par le champ électrique appliqué à une suspension de micro-organismes dans une huile diélectrique permet de conclure à la présence ou à l'absence d'activité biologique. Le procédé peut, lorsqu'il est suivi d'une étape de récupération des micro-organismes et de comptage, être utilisé pour déterminer la viabilité des micro-organismes après l'application d'un champ électrique.

La détermination simple et rapide de l'activité biologique de micro-organismes est essentielle dans de nombreux domaines comme les contrôles de pollution, l'assurance qualité et l'analyse clinique. Dans les domaines de la recherche et de l'industrie agroalimentaire, les micro-organismes tels que les bactéries et les levures sont des outils de plus en plus utilisés. L'utilisation et l'efficacité des différents processus biochimiques mis en jeu dépendent de la viabilité de ces micro-organismes.

Dans cet objectif, la technique standard consiste à mettre en culture un échantillon dans des conditions particulières et dans un milieu gélosé approprié du type agar-agar. La reproduction effective des éléments prélevés, observée visuellement ou au microscope accompagnée d'un comptage, permet de conclure à l'existence d'une activité biologique. Dans ce cas, la souche d'origine est déclarée vivante. Dans le cas contraire, on conclut à son inactivité biologique.

Ce procédé présente de nombreux inconvénients et en particulier le temps nécessaire à l'obtention du résultat. Dans le meilleur des cas, 18 heures sont nécessaires pour la formation de colonies visibles.

Le brevet US 4321322 décrit un procédé de détermination de l'existence de l'activité biologique de micro-organismes par application d'un champ électrique à des micro-organismes en suspension dans un milieu aqueux.

Le brevet US 5824494 décrit notamment un dispositif pour la détermination du nombre de micro-organismes dans un échantillon liquide.

Par ailleurs, le brevet EP 0543090 propose une méthode analytique basée sur la mesure directe de l'activité métabolique de bactéries par utilisation d'une cellule bioélectrochimique. Plusieurs aspects du système de détection bioélectrochimique sont décrits dans le brevet EP 0221663 concernant une méthode et un instrument pour la mesure de l'activité microbienne dans une cellule bioélectrochimique, EP 0219247 concernant la cellule bioélectrochimique et ces électrodes, EP 0238322 concernant les médiateurs bioélectrochimiques et EP 0352138 concernant les électrodes perfectionnées utilisées dans les cellules bioélectrochimiques. Certains de ces procédés comportent une étape précédant l'analyse dans laquelle les bactéries sont concentrées sur un filtre, élément essentiel du dispositif. D'autres utilisent une électrode à la configuration particulière et de composition spéciale en carbone poreux ou en métaux nobles aux coûts élevés.

Quels que soient les systèmes décrits par ces brevets, l'utilisation d'un médiateur chimique est essentielle. Ce médiateur est un composé organique tel que la 1,4-benzoquinone, qui est dissous dans le milieu et qui sert au transfert d'électrons entre les électrodes et les micro-organismes. Il génère, au contact des bactéries, une réponse détectée et mesurée par les électrodes de mesure. En fonction du médiateur, de sa concentration et de son degré d'oxydation, les réponses mesurées varient nettement, ce qui rend difficile l'interprétation et la comparaison des résultats. De plus, les éventuelles substances ou éléments contaminants capables d'échanger des électrons avec le médiateur sont la cause de nombreux artefacts. Enfin, la présence du médiateur et d'un filtre permet difficilement d'envisager une récupération ultérieure des micro-organismes étudiés.

Lors de l'utilisation d'une cellule bioélectrochimique, les micro-organismes sont soumis à des champs électriques en milieu aqueux et plus particulièrement, dans un tampon aux concentrations ioniques précises. Les courants électriques de type alternatif en haute Séquence produisent des champs électriques alternatifs ou tournants. De telles conditions ne permettent pas d'appliquer des tensions supérieures à quelques volts pendant des durées supérieures à quelques secondes. En utilisant le courant continu, la tension électrique obtenue par des décharges de condensateurs a toujours un caractère transitoire ou impulsionnel. On ne peut la maintenir à une valeur élevée de plusieurs kilovolts que pendant des durées relativement courtes inférieures à une seconde; ceci ne permet, par conséquent, que des mesures discontinues ou par paliers.

Le but de l'invention est de remédier aux inconvénients ci-dessus en proposant un procédé rapide de détermination de l'état d'une culture de micro-organismes adaptable à différentes espèces de micro-organismes.

La présente invention a également pour but de fournir un dispositif adapté à une mise en oeuvre simple et peu coûteuse du procédé précité. Un autre but est de permettre la récupération des micro-organismes étudiés après l'application d'un champ électrique selon le procédé de l'invention.

Pour ce faire, l'invention a pour objet un procédé de détermination de l'existence d'une activité biologique de micro-organismes par l'application d'un champ électrique caractérisé en ce qu'il comprend l'application d'un champ électrique à un milieu diélectrique constitué par des micro-organismes en suspension dans une huile diélectrique et la mesure d'un courant de fuite caractéristique lorsque les micro-organismes sont vivants.

Pour réaliser la suspension cellulaire, les micro-organismes sont, au préalable, prélevés à la surface d'un milieu de culture gélosé puis introduits dans une huile diélectrique. L'huile diélectrique peut être un mélange d'huiles ou une huile minérale ou organique et, de préférence, une huile de silicone. Le mélange micro-organismes/huile diélectrique peut être homogénéisé au moyen d'une sonde à ultrasons.

Le procédé selon l'invention, consiste à appliquer progressivement, à la suspension diélectrique, un champ élecuique depuis la valeur zéro jusqu'à la valeur maximale choisie. L'application du champ électrique crée au sein du milieu qui contient les micro-organismes et l'huile diélectrique, un courant de fuite. Lorsque les micro-organismes sont vivants, les variations de l'intensité du courant de fuite en fonction du champ électrique appliqué sont caractéristiques et permettent de conclure à l'existence d'une activité biologique. Quel que soit le stress consécutif à l'application du champ électrique, il y a apparition d'un pic caractéristique de la viabilité des micro-organismes étudiés. Lorsque les micro-organismes sont morts, on observe, au contraire, une variation de l'intensité constante en fonction du champ électrique appliqué.

L'invention concerne aussi un dispositif de détermination de l'existence d'une activité biologique de micro-organismes caractérisé en ce qu'il comprend un conteneur équipé d'électrodes dans lequel est placée la suspension de micro-organismes dans une huile diélectrique, des moyens de génération d'un champ électrique entre deux ou plusieurs électrodes, des moyens de mesure et des moyens d'enregistrement du courant de fuite induit. Le conteneur dans lequel est introduite la suspension de micro-organismes est équipé d'électrodes métalliques.

Selon un autre mode de réalisation de l'invention, le conteneur dans lequel est introduite ladite suspension est constitué par une plaque isolante munie d'un espace inter-électrodes. De préférence, la tension du courant électrique entre les électrodes varie de 0 et 250 Volts par millimètre.

L'application du champ électrique, selon le procédé de l'invention, peut être suivie d'une étape de transfert en milieu aqueux puis d'une étape de comptage. Cette application du procédé constitue un moyen simple et efficace de sélection des micro-organismes ayant survécu au champ électrique.

Les caractétistiques et avantages de l'invention apparaîtront dans la description détaillée non limitative des exemples ci-dessous.

La figure 1 représente le conteneur 1 contenant un enrobage 4 qui maintient les électrodes 2 entre lesquelles est introduite la suspension de micro-organismes 3.

La figure 2 représente une variante du conteneur constitué par une boîte de Pétri 5 munie d'électrodes 2 entre lesquelles est placée la suspension de micro-organismes 3 sur une lamelle 6 dans l'espace inter-électrodes 7.

La figure 3 représente une courbe des variations du courant de fuite en fonction du champ électrique appliqué lorsque les micro-organismes sont morts.

La figure 4 représente une courbe des variations de l'intensité du courant de fuite en fonction du champ électrique appliqué allant de 0 à 250 V/mm lorsque les micro-organismes sont vivants.

La figure 5 représente une courbe des variations de l'intensité du courant de fuite en fonction du temps lorsque la tension est montée par paliers de 2,5 V/mm toutes les 15 secondes jusqu'à une tension finale de 25 V/mm symbolisée par la flèche puis maintenue à cette valeur.

La figure 6 représente une courbe des variations de l'intensité du courant de fuite en fonction du temps lorsque la tension est montée par paliers de 5 V/mm toutes les 15 secondes jusqu'à une tension finale de 50 V/mm symbolisée par la flèche puis maintenue à cette valeur.

La figure 7 représente une courbe des variations de l'intensité du courant de fuite en fonction du temps lorsque la tension est montée par paliers de 10 V/mm toutes les 15 secondes jusqu'à une tension finale de 250 V/mm symbolisée par la flèche puis maintenue à cette valeur.

### Exemple 1 :

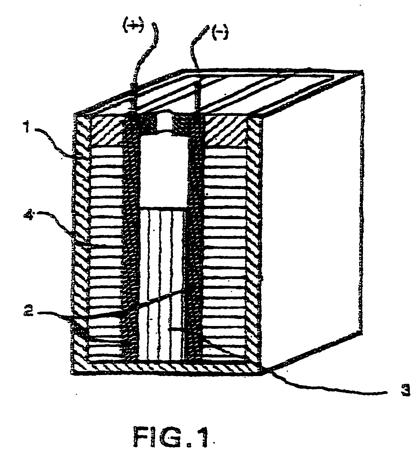

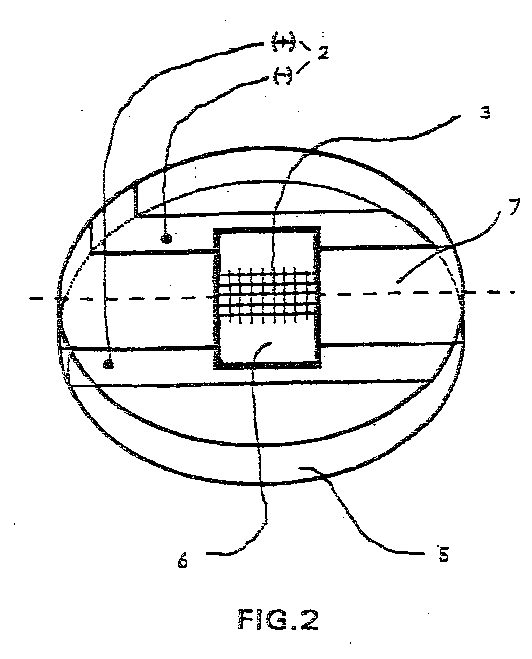

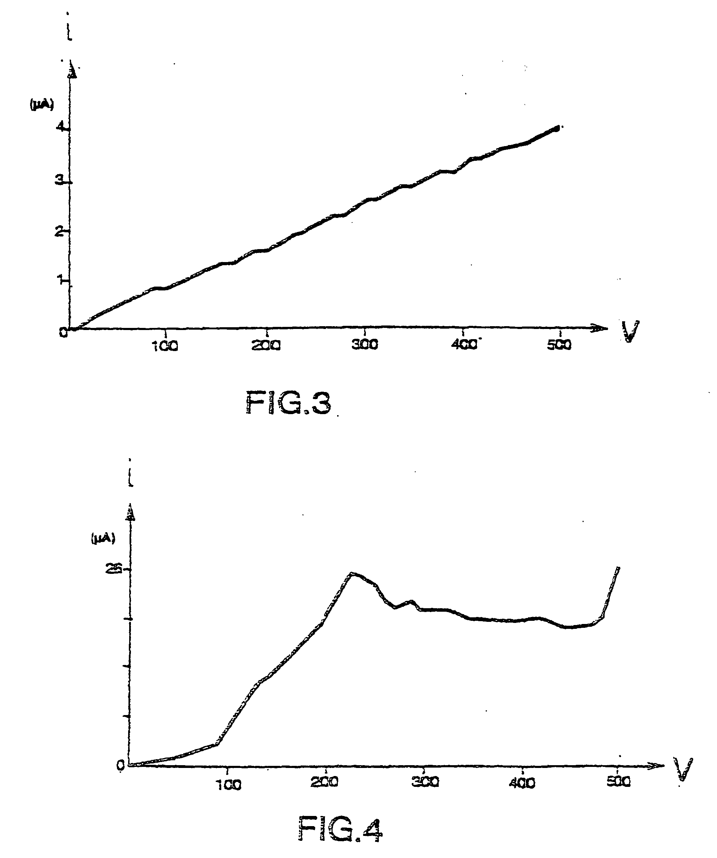

Lorsque les micro-organismes étudiés proviennent d'un milieu de culture gélose, ils sont prélevés en surface à l'aide d'un racloir et introduits dans un microtube Eppendorf contenant 1 ml d'huile diélectrique du type huile de silicone. On introduit une sonde à ultrasons dans le microtube. Les ultrasons à 25 W pour une fréquence de 20 kHz sont appliqués au mélange pendant 30 secondes en alternance avec 2 à 3 phases de repos de 5 secondes, afin d'obtenir une suspension homogène ; les agrégats cellulaires et la gélose éventuellement présents sont éliminés par centrifugation à 4000 tours/min pendant 30 secondes à 4°C. La suspension homogène est ensuite introduite dans l'un des conteneurs décrits sur les figures 1 et 2. L'écartement entre les électrodes 2 est de 1,8 mm pour le dispositif représenté sur la figure 1 et 3 mm pour celui de la figure 2. Une tension continue variant progressivement entre 0 à 250 Volts par mm est fournie par un générateur et appliquée progressivement à la suspension. En parallèle, un ampèremètre enregistre le courant de fuite induit.

D'après le tracé de la courbe représentative de la variation de l'intensité du courant de fuite i en microampères en fonction du champ électrique appliqué V en volts entre les électrodes 2, on peut déterminer immédiatement et simplement si les micro-organismes sont vivants ou morts.

Lorsque les micro-organismes sont morts, l'allure de la courbe représentée sur la figure 3 et obtenue avec le dispositif de la figure 1 est linéaire. En revanche, lorsqu'ils sont vivants, l'allure de la courbe représentée sur la figure 4 et obtenue avec le dispositif de la figure 1 est très différente. Le courant de fuite augmente brutalement pour de faibles tensions, passe par un maximum pour une tension d'environ 220 volts puis redescend brutalement pour se stabiliser à partir d'une valeur de l'ordre de 300 volts.

### Exemple 2 :

Les micro-organismes étudiés provenant d'un milieu de culture gélosé sont prélevés en surface à l'aide d'un racloir et introduits dans deux microtubes Eppendorf contenant chacun 1 ml d'huile diélectrique du type huile de silicone. On introduit une sonde à ultrasons dans les microtubes appelés microtube expérimental et microtube témoin. Les ultrasons à 25 W pour une Séquence de 20 kHz sont appliqués au mélange pendant 30 secondes en alternance avec 2 à 3 phases de repos de 5 secondes, afin d'obtenir une suspension homogène; les agrégats cellulaires et la gélose éventuellement présents sont éliminés par centrifugation à 4000 tours/min pendant 30 secondes à 4°C.

Après introduction de la suspension homogène contenue dans le microtube expérimental dans l'un des conteneurs décrit sur les figures 1 et 2, le champ électrique est appliqué. Ainsi, un champ électrique aux caractéristiques qualitatives et quantitatives préalablement déterminées selon le type d'études menées et selon les micro-organismes étudiés est appliqué à l'une des suspensions 3 pendant une durée déterminée. Le milieu est transféré dans un microtube Eppendorf expérimental 2.

Les deux microtubes sont ensuite centrifugés à 10000 tours/min pendant 30 secondes à 4°C afin de récupérer les culots. On retire l'huile de silicone en renversant les microtubes Eppendorf. On ajoute 1 ml de solution tampon phosphate de pH 7 à 0,05M dans les microtubes témoin et expérimental 2 et chacun des mélanges est homogénéisé par sonication à 25 W pour une fréquence de 20 kHz pendant 5 secondes. Chaque suspension est versée dans un tube cotonné contenant 9 ml de milieu de culture approprié aux micro-organismes étudiés.

Les cellules bactériennes sont revivifiées consécutivement à l'agitation pendant 3 heures à 150 tours/min des tubes cotonnés témoin et expérimental. 1 ml de cette première dilution est prélevé dans chaque tube et transféré dans un tube à hémolyse afin de mesurer la densité optique de chaque suspension cellulaire. On rajoute aux deux prélèvements une quantité suffisante de tampon phosphate de pH 7 à 0,05M pour obtenir une densité optique finale comprise entre 0 et 0,4. Connaissant la densité optique du témoin et de l'expérimental, on déduit par comparaison avec une courbe étalon obtenue par comptage à la cellule de Thoma, le nombre de cellules présentes dans chaque tube.

On prépare une gamme de dilutions de 10 en 10 pour chaque tube. Pour chaque dilution, on ensemence 3 boîtes de Pétri contenant du milieu gélosé avec 0,1 ml de suspension cellulaire. Après 72 heures d'incubation à 30°C, on dénombre les colonies formées sur chaque boîte. On détermine, alors, le pourcentage de cellules vivantes suite à l'application du champ électrique en effectuant le rapport du nombre de cellules présentes dans le tube expérimental au nombre de cellules présentes dans le tube témoin.

### Exemple 3:

On opère comme dans l'exemple 1.

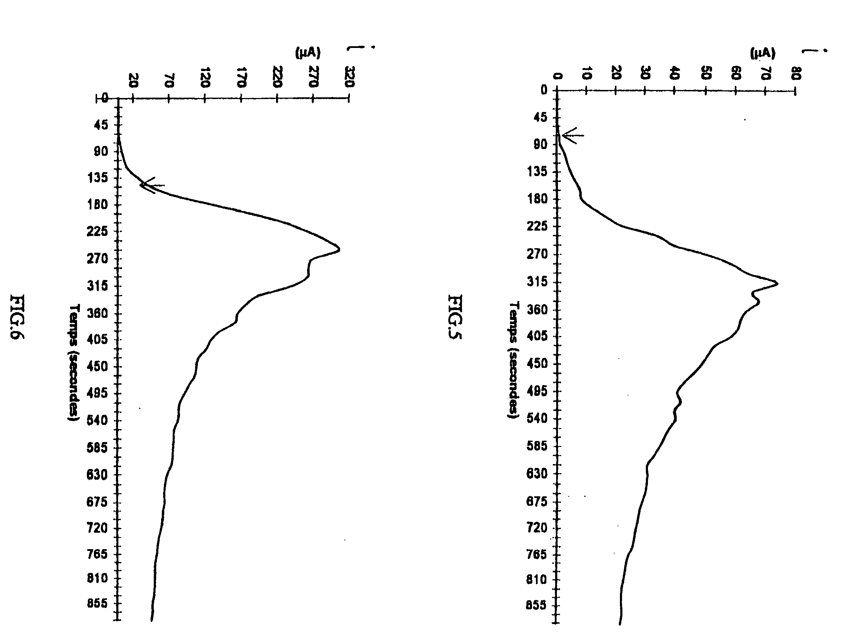

L'intensité du champ électrique appliqué à la suspension 3 est augmentée par paliers de 2,5 V/mm toutes les 15 secondes jusqu'à obtention d'une tension de 25 V/mm. L'arrêt de la montée en tension est symbolisé sur la figure 5 par la flèche. Le temps nécessaire pour cette montée en tension est de 1 minute et 15 secondes. Ensuite, la tension est maintenue à cette valeur pendant 13 minutes et 45 secondes, ce qui conduit à une durée totale d'expérience de 15 minutes. En parallèle, un ampèremètre enregistre le courant de fuite induit.

Dès que l'intensité du champ électrique ne croît plus, l'intensité du courant de fuite augmente, dans un premier temps linéairement pendant 100 secondes puis une cassure survient et l'intensité du courant de fuite croît fortement pour atteindre une valeur maximale de plus de 70 µA au bout de 330 secondes. Pendant le reste du temps durant lequel le champ électrique est appliqué à la suspension cellulaire 3, l'intensité du courant de fuite décroît jusqu'à une valeur finale avoisinant 20 µA. La régularité de cette décroissance est altérée par la présence de pics secondaires de faible intensité.

### Exemple 4 :

On opère comme dans l'exemple 1.

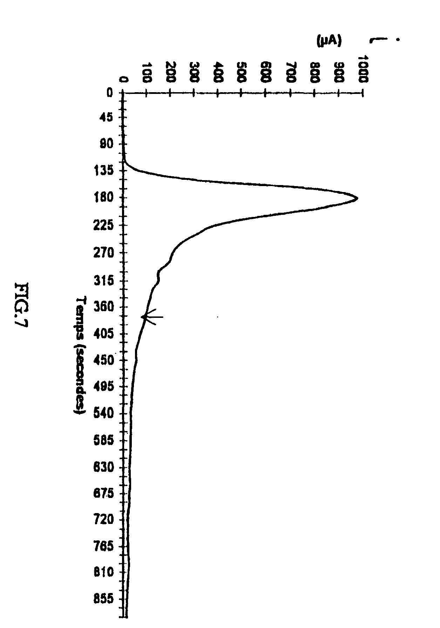

L'intensité du champ électrique appliqué à la suspension 3 est augmentée par paliers de 5 V/mm toutes les 15 secondes jusqu'à obtention d'une tension de 50 V/mm. L'arrêt de la montée en tension est symbolisé sur la figure 6 par la flèche. Le temps nécessaire pour cette montée en tension est de 2 minutes et 30 secondes. Ensuite, la tension est maintenue à cette valeur pendant 12 minutes et 30 secondes, ce qui conduit à une durée totale d'expérience de 15 minutes. En parallèle, un ampèremètre enregistre le courant de fuite induit.

Après une phase de croissance faible pendant la première minute, l'intensité du courant de fuite commence à augmenter rapidement Il s'agit de l'amorce du pic d'intensité de courant de fuite survenant après l'arrêt de la montée en tension. La valeur maximale de l'intensité du courant de fuite enregistrée après 4 minutes et 30 secondes est de 306 µA. Pendant le reste du temps durant lequel le champ électrique est appliqué à la suspension cellulaire 3, l'intensité du courant de fuite décroît jusqu'à une valeur finale avoisinant 47 µA. La régularité de cette décroissance est altérée par la présence de pics secondaires de faible intensité.

### Exemple 5 :

On opère comme dans l'exemple 1.

L'intensité du champ électrique appliqué à la suspension 3 est augmentée par paliers de 10 V/mm toutes les 15 secondes jusqu'à obtention d'une tension de 250 V/mm. L'arrêt de la montée en tension est symbolisé sur la figure 7 par la flèche. Le temps nécessaire pour cette montée en tension est de 6 minutes et 15 secondes. Ensuite, la tension est maintenue à cette valeur pendant 8 minutes et 45 secondes, ce qui conduit à une durée totale d'expérience de 15 minutes. En parallèle, un ampèremètre enregistre le courant de fuite induit.

L'intensité du courant de fuite croît faiblement pendant les deux premières minutes de la montée en tension, soit jusqu'à ce que l'intensité du champ électrique atteigne 80 V/mm. Dans la minute suivante, soit entre 90 et 130 V/mm, une très forte augmentation de l'intensité du courant de fuite est enregistrée. Au terme de cette accroissement, la valeur de l'intensité du courant de fuite est de 97,6 V/mm. Ensuite, l'intensité du courant de fuite décroît de manière quasi exponentielle jusqu'à la fin de l'application de la tension et atteint une valeur finale de 15 µA. La régularité de cette décroissance est altérée par la présence de pics secondaires de faible intensité.

Le procédé selon l'invention permet de déterminer l'état de la culture bactérienne en un temps inférieur à l'heure. Il évite donc des manipulations coûteuses et la participation d'un personnel compétent pour exécuter les observations visuelles et le comptage des éléments repiqués dans un milieu nourricier. De plus, du fait de la faible durée du contrôle, le développement des contaminants est négligeable. Il n'est donc pas nécessaire de travailler en atmosphère stérile ou dans des conditions d'asepsie rigoureuses.

D'autres avantages tels que l'absence de médiateur spécifique et l'utilisation d'électrodes classiques permettent d'obtenir des résultats précis pour un faible coût.

Le procédé présente également l'avantage d'appliquer des champs électriques croissants de forte intensité sur de longues périodes. Les mesures ainsi effectuées en continu aboutissent, par conséquent, à des résultats plus cohérents dont l'interprétation sera plus fiable.

L'invention présente également l'avantage de permettre une récupération pour une éventuelle utilisation ultérieure des micro-organismes qui auraient survécu à l'application du champ électrique.

## Revendications

1. Procédé de détermination de l'existence d'une activité biologique de micro-organismes par application d'un champ électrique, **caractérisé en ce qu'**il comprend la mise en suspension des micro-organismes dans une huile diélectrique, l'application d'un champ électrique à ladite suspension et la mesure des variations de l'intensité du courant de fuite induit en fonction du champ électrique appliqué.

2. Procédé selon la revendication 1, **caractérisé en ce que** les micro-organismes sont prélevés sur un milieu de culture gélosé.

3. Procédé selon la revendication 1, **caractérisé en ce que** la suspension de micro-organismes est obtenue par application d'ultrasons au mélange constitué par l'huile diélectrique et les micro-organismes.

4. Procédé selon la revendication 1, **caractérisé en ce que** l'huile diélectrique est une huile minérale ou organique ou un mélange de celles-ci.

5. Procédé selon la revendication 4, **caractérisé en ce** l'huile diélectrique est une huile de silicone.

6. Procédé selon la revendication 1, **caractérisé en ce que** le champ électrique appliqué à la suspension des micro-organismes varie de 0 à 250 volts par millimètre.

7. Procédé selon l'une quelconque des revendications 1 à 6, **caractérisé en ce** postérieurement à la mesure des variations de l'intensité du courant de fuite induit, les micro-organismes sont transférés dans un milieu aqueux et comptés.

## Patentansprüche

1. Verfahren zur Feststellung des Vorhandenseins einer biologischen Aktivität von Mikroorganismen durch Anlegen eines elektrischen Feldes, **dadurch gekennzeichnet, daß** es umfaßt: das Suspendieren von Mikroorganismen in einem dielektrischen Öl, das Anlegen eines elektrischen Feldes an die Suspension und das Messen der Änderungen der Stärke des in Abhängigkeit des angelegten elektrischen Feldes induzierten Verluststroms.

2. Verfahren nach Anspruch 1, **dadurch gekennzeichnet, daß** die Mikroorganismen von einem Agar-Kulturmedium abgenommen werden.

3. Verfahren nach Anspruch 1, **dadurch gekennzeichnet, daß** die Mikroorganismen-Suspension durch Ultraschallbehandlung eines Gemischs aus dem dielektrischen Öl und den Mikroorganismen erhalten wird.

4. Verfahren nach Anspruch 1, **dadurch gekennzeichnet, daß** das dielektrische Öl ein Mineralöl oder ein organisches Öl oder ein Gemisch. davon ist.

5. Verfahren nach Anspruch 4, **dadurch gekennzeichnet, daß** das dielektrische Öl ein Silikonöl ist.

6. Verfahren nach Anspruch 1, **dadurch gekennzeichnet, daß** das an die Suspension der Mikroorganismen angelegte elektrische Feld zwischen 0 und 250 V/mm beträgt.

7. Verfahren nach einem der Ansprüche 1 bis 6, **dadurch gekennzeichnet, daß** die Mikroorganismen nach dem Messen der Änderungen der Stärke des induzierten Verluststroms in ein wäßriges Medium übergeführt und gezählt werden.

## Claims

1. A process for determining the existence of biological activity of micro-organisms by application of an electric field, **characterised in that** said process comprises suspending micro-organisms in a dielectric oil, applying an electric field to said suspension, and measuring the variations in the intensity of the induced leakage current as a function of the applied electric field.

2. Process according to Claim 1, **characterised in that** the micro-organisms are taken from an agar culture medium.

3. Process according to Claim 1, **characterised in that** the suspension of micro-organisms is obtained by application of ultrasound to the mixture constituted by the dielectric oil and the micro-organisms.

4. Process according to Claim 1, **characterised in that** the dielectric oil is a mineral oil or an organic oil or a mixture thereof.

5. Process according to Claim 4, **characterised in that** the dielectric oil is a silicone oil.

6. Process according to Claim 1, **characterised in that** the electric field applied to the suspension of the micro-organisms varies from 0 volts per millimetre to 250 volts per millimetre.

7. Process according to any one of Claims 1 to 6, **characterised in that** subsequent to the measurement of the variations in the intensity of the induced leakage current the micro-organisms are transferred to an aqueous medium and counted.
